# EUROPÄISCHE PATENTSCHRIFT

(11) **EP 0 625 504 B1**
(45) Veröffentlichungstag und Bekanntmachung des Hinweises auf die Patenterteilung: **27.08.1997**
(21) Anmeldenummer: 93107734.1
(22) Anmeldetag: 12.05.1993
(51) Int. Cl.: C07C 231/22, C07C 233/73

(54) **Stabile und kristalline Form von Bezafibrat**
Stable and crystaline form of Bezafibrate
Forme stabile et crystalline de Bezafibrate

(43) Veröffentlichungstag der Anmeldung: 23.11.1994
(73) Patentinhaber: HEUMANN PHARMA GmbH, D-90478 Nürnberg (DE)
(72) Erfinder: Mörsdorf, Peter Dr. Dipl. Chem., D-90579 Langenzenn (DE); Maier, Peter Chemikant, D-90537 Feucht (DE); Schmitt, Josef Chemieingenieur, D-91522 Ansbach (DE); Ahrens, Kurt-Henning Dr. Apotheker, D-90403 Nürnberg (DE)
(74) Vertreter: Kraus, Walter, Dr.

(56) Entgegenhaltungen:
- EP-A- 0 245 156
- DE-A- 2 149 070
- DE-A- 2 920 413
- GB-A- 2 021 575

## Beschreibung

Die Verbindung 2-[4-[2-(4-Chlorbenzoylamino)ethyl]phenoxy]-2-methylpropionsäure der Formel I 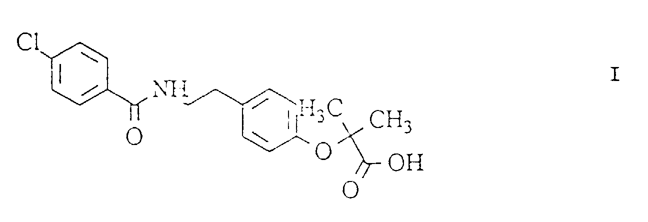 mit dem internationalen Freinamen Bezafibrat und ihre Herstellung sind bereits aus der DE-PS 21 49 070 bekannt. Bezafibrat ist ein wertvolles Arzneimittel, das eine starke Senkung der Serumlipide und des Cholesterinspiegels bewirkt, und das deswegen zur Behandlung von Arteriosklerose verwendet werden kann. Weitere Herstellungsverfahren für Bezafibrat werden in der DE-PS 29 20 413 und der US-PS 4,739,101 beschrieben. Bei den in diesen Druckschriften beschriebenen Synthesen wird das als Endprodukt erhaltene Bezafibrat aus Aceton oder Wasser kristallisiert, wobei je nach Herstellungsweise Bezafibrat-Endprodukte mit Schmelzpunkten von 178°C bis 186°C erhalten werden. Hinweise auf das Vorliegen von polymorphen Formen finden sich in den genannten Druckschriften nicht.

Die Röntgenstrukturanalyse der bekannten Form des Bezafibrats hat ergeben, daß die nadelförmigen Kristalle des bekannten Bezafibrats dem orthorhombischen Kristallsystem zuzuordnen sind [vgl. K. Djinovic et al., Acta Crystallographica C 45, S. 772-775, (1989)]. Die bekannte orthorhombische Kristallform des Bezafibrats ist aber mit dem Nachteil behaftet, daß sie in Form von feinen, verfilzten Nadeln kristallisiert ist, die galenisch schlecht zu verarbeiten sind. Ein weiterer Nachteil der bekannten Kristallform des Bezafibrats ist die geringe Schüttdichte von 0,26 g pro Milliliter.

Der Erfindung liegt daher die Aufgabe zugrunde, eine neue Kristallform des Bezafibrats bereitzustellen, die von den obigen Nachteilen frei ist und die in Form von Kristallen anfällt, die galenisch besser zu verarbeiten sind.

Diese Aufgabe wird erfindungsgemäß durch eine neue, stabile und kristalline Form des Bezafibrats, die nachfolgend als β-Form bezeichnet wird, gelöst, die durch ein Infrarotspektrum in Kaliumbromid mit Absorptionsbanden bei folgenden Wellenzahlen gekennzeichnet ist:

| | |
|---|---|
| 3389 cm⁻¹ | 847 cm⁻¹ |
| 1740 cm⁻¹ | 821 cm⁻¹ |
| 1328 cm⁻¹ | 618 cm⁻¹ |
| | 606 cm⁻¹ |

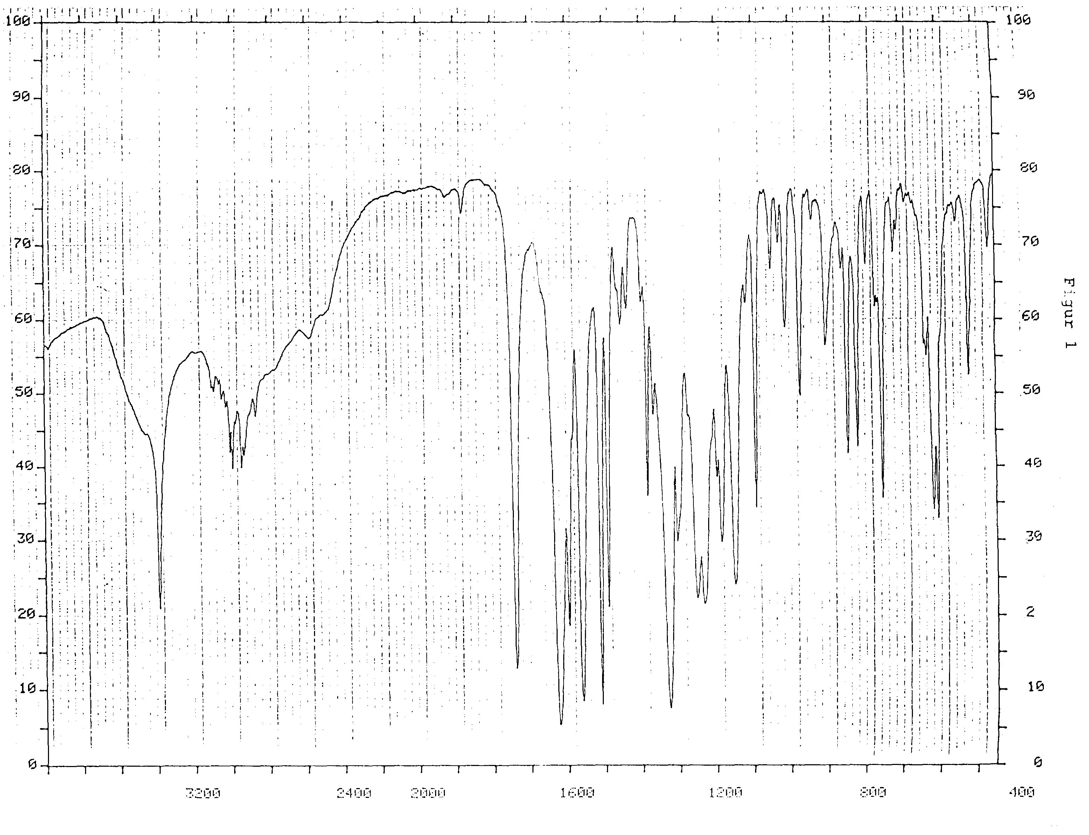

Eine einfache und sichere Identifizierung und Charakterisierung der erfindungsgemäßen β-Form von Bezafibrat ist anhand des FT-IR-Spektrums möglich. Die Figur 1 zeigt das FT-IR-Spektrum der erfindungsgemäßen β-Form, aufgenommen als Kaliumbromid-Pressling. Insbesondere im Fingerprint-Bereich zwischen 900 und 600 cm⁻¹ zeigt die erfindungsgemäße β-Form charakteristische, starke Banden. Daraus ergibt sich, daß es sich bei der erfindungsgemäßen β-Form des Bezafibrats um eine neue, bislang noch nicht beschriebene Form dieser Verbindung handelt.

Die erfindungsgemäße β-Form von Bezafibrat unterscheidet sich von der bisher bekannten Form über das IR-Spektrum hinaus noch in einer Reihe von weiteren Eigenschaften. Diese Eigenschaften können daher ebenfalls zur Unterscheidung von der bisher bekannten orthorhombischen Form herangezogen werden.

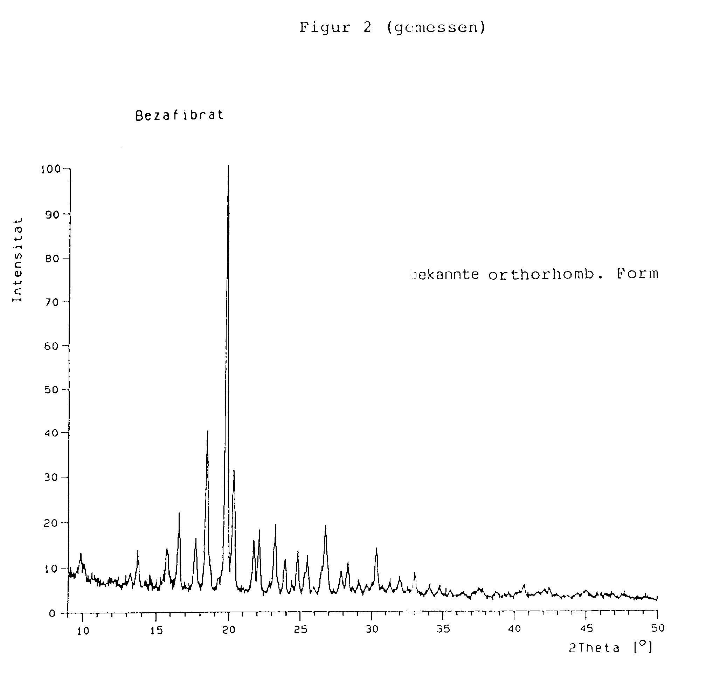

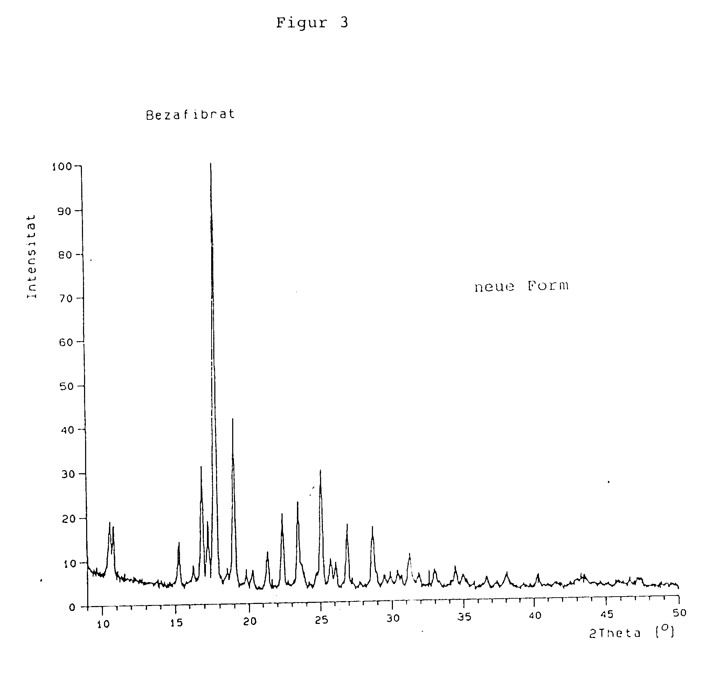

Die Figuren 2 und 3 geben als Ergebnis röntgenographischer Untersuchungen die Pulverdiffraktogramme der bekannten orthorhombischen Form und der erfindungsgemäßen β-Form wieder. Die Pulverdiagramme beider Proben wurden unter gleichen Bedingungen auf einen Pulverdiffraktometer Philips PW 1050/25 in Plexiglasträgern mit 0,3 mm Schichtdicke aufgenommen, unter Cu-K_{α}-Strahlung und unter Verwendung eines Ni-Filters. Die Diagramme wurden im Beugungswinkelbereich von 2 Θ : 9 - 50° mit einer Schrittweite von 0,02° und einer Meßzeit von 1 Sekunde pro Schritt gemessen.

Die gefundenen Reflexlagen und ihre relativen Intensitäten sind in Tabelle 1 für die bekannte orthorhombische Form und in Tabelle 2 für die neue Form zusammengestellt.

**Tabelle 1**

| **Röntgen-Pulverdiagramm von Bezafibrat** (orthorhombische, bekannte Form) | | | | | | | |
|---|---|---|---|---|---|---|---|
| Reflexliste (auf CuKα₁ bezogen) | | | | | | | |
| Nr. | d [Å] | Iᵣₑₗ | 2θ | Nr. | d [Å] | Iᵣₑₗ | 2θ |
| | | | | | | | |
| 1 | 9.6771 | 13 | 9.131 | 44 | 2.4192 | 15 | 37.133 |
| 2 | 9.1872 | 18 | 9.619 | 45 | 2.3963 | 24 | 37.501 |
| 3 | 9.0087 | 44 | 9.810 | 46 | 2.3225 | 15 | 38.741 |
| 4 | 8.7767 | 31 | 10.070 | 47 | 2.2884 | 10 | 39.341 |
| 5 | 7.3651 | 7 | 12.007 | 48 | 2.2741 | 12 | 39.598 |
| 6 | 7.2563 | 10 | 12.188 | 49 | 2.2404 | 14 | 40.220 |
| 7 | 6.6942 | 28 | 13.215 | 50 | 2.2175 | 32 | 40.654 |
| 8 | 6.4468 | 69 | 13.725 | 51 | 2.1693 | 15 | 41.599 |
| 9 | 6.2160 | 9 | 14.237 | 52 | 2.1460 | 21 | 42.071 |
| 10 | 6.0560 | 13 | 14.615 | 53 | 2.1296 | 24 | 42.411 |
| 11 | 5.7098 | 21 | 15.507 | 54 | 2.1053 | 9 | 42.925 |
| 12 | 5.6204 | 93 | 15.755 | 55 | 2.0190 | 16 | 44.855 |
| 13 | 5.3510 | 153 | 16.553 | 56 | 1.9807 | 10 | 45.773 |
| 14 | 5.0128 | 107 | 17.679 | 57 | 1.9584 | 10 | 46.323 |
| 15 | 4.8080 | 365 | 18.438 | 58 | 1.9396 | 12 | 46.800 |
| 16 | 4.5990 | 26 | 19.284 | 59 | 1.9118 | 9 | 47.522 |
| 17 | 4.4770 | 1000 | 19.815 | 60 | 1.8176 | 9 | 50.150 |
| 18 | 4.3656 | 282 | 20.326 | | | | |
| 19 | 4.0840 | 115 | 21.744 | | | | |
| 20 | 4.0164 | 131 | 22.114 | | | | |
| 21 | 3.8913 | 23 | 22.835 | | | | |
| 22 | 3.8265 | 141 | 23.227 | | | | |
| 23 | 3.7203 | 77 | 23.899 | | | | |
| 24 | 3.6422 | 18 | 24.420 | | | | |
| 25 | 3.5878 | 88 | 24.795 | | | | |
| 26 | 3.4928 | 82 | 25.482 | | | | |
| 27 | 3.4339 | 11 | 25.926 | | | | |
| 28 | 3.3313 | 160 | 26.739 | | | | |
| 29 | 3.2039 | 47 | 27.824 | | | | |
| 30 | 3.1499 | 69 | 28.310 | | | | |
| 31 | 3.1132 | 13 | 28.651 | | | | |
| 32 | 3.0667 | 22 | 29.095 | | | | |
| 33 | 3.0107 | 18 | 29.648 | | | | |
| 34 | 2.9462 | 106 | 30.313 | | | | |
| 35 | 2.9081 | 16 | 30.719 | | | | |
| 36 | 2.8586 | 22 | 31.266 | | | | |
| 37 | 2.7979 | 36 | 31.961 | | | | |
| 38 | 2.7505 | 8 | 32.528 | | | | |
| 39 | 2.7098 | 46 | 33.030 | | | | |
| 40 | 2.6308 | 21 | 34.051 | | | | |
| 41 | 2.5796 | 17 | 34.748 | | | | |
| 42 | 2.5271 | 12 | 35.494 | | | | |
| 43 | 2.4641 | 13 | 36.434 | | | | |

**Tabelle 2**

| **Röntgen-Pulverdiagramm von Bezafibrat** (erfindungsgemäße β-Form) | | | |
|---|---|---|---|
| Nr. | d [Å] | Iᵣₑₗ | 2θ |
| | | | |
| 1 | 8.3768 | 133 | 10.552 |
| 2 | 8.1896 | 115 | 10.794 |
| 3 | 5.8004 | 96 | 15.263 |
| 4 | 5.4492 | 49 | 16.253 |
| 5 | 5.2592 | 285 | 16.845 |
| 6 | 5.1410 | 154 | 17.235 |
| 7 | 4.9926 | 1000 | 17.751 |
| 8 | 4.7903 | 26 | 18.507 |
| 9 | 4.6688 | 396 | 18.993 |
| 10 | 4.4792 | 30 | 19.805 |
| 11 | 4.3819 | 41 | 20.249 |
| 12 | 4.1699 | 90 | 21.291 |
| 13 | 3.9759 | 181 | 22.342 |
| 14 | 3.7908 | 206 | 23.449 |
| 15 | 3.5965 | 36 | 24.735 |
| 16 | 3.5493 | 293 | 25.069 |
| 17 | 3.4620 | 65 | 25.712 |
| 18 | 3.4151 | 59 | 26.071 |
| 19 | 3.3112 | 148 | 26.904 |
| 20 | 3.1100 | 152 | 28.682 |
| 21 | 3.0303 | 27 | 29.452 |
| 22 | 2.9882 | 30 | 29.877 |
| 23 | 2.9404 | 35 | 30.374 |
| 24 | 2.8626 | 81 | 31.220 |
| 25 | 2.8053 | 27 | 31.875 |
| 26 | 2.7140 | 39 | 32.977 |
| 27 | 2.6044 | 44 | 34.408 |
| 28 | 2.5651 | 31 | 34.952 |
| 29 | 2.4527 | 31 | 36.608 |
| 30 | 2.4104 | 15 | 37.274 |
| 31 | 2.3625 | 41 | 38.059 |
| 32 | 2.2359 | 31 | 40.305 |
| 33 | 2.1757 | 15 | 41.470 |
| 34 | 2.1243 | 13 | 42.522 |
| 35 | 2.1030 | 22 | 42.973 |
| 36 | 2.0893 | 26 | 43.270 |
| 37 | 2.0788 | 30 | 43.499 |
| 38 | 2.0133 | 8 | 44.991 |
| 39 | 1.9787 | 17 | 45.822 |
| 40 | 1.9465 | 16 | 46.625 |
| 41 | 1.9260 | 21 | 47.149 |

Charakteristische Reflexe mit hoher relativer Intensität treten im Pulverdiagramm der erfindungsgemäßen β-Form bei Beugungswinkeln von 16,8°, 17,7°, 19,0° und 25,1° auf. Dagegen fehlen die für die bekannte orthorhombische Form charakteristischen Reflexe bei 18,5° und 19,8°.

Die erfindungsgemäße β-Form hat aufgrund der vergleichbaren Reflexbreiten und Intensitäten eine ähnlich gute Kristallinität, zeigt aber in den Reflexlagen und der Intensitätsverteilung keinerlei Ähnlichkeit mit der bekannten orthorhombischen Form. Es handelt sich bei der neuen Form also um eine deutlich unterschiedliche Kristallstruktur. Eine Beimischung der bekannten orthorhombischen Form ist in der neuen Form röntgenographisch auszuschließen.

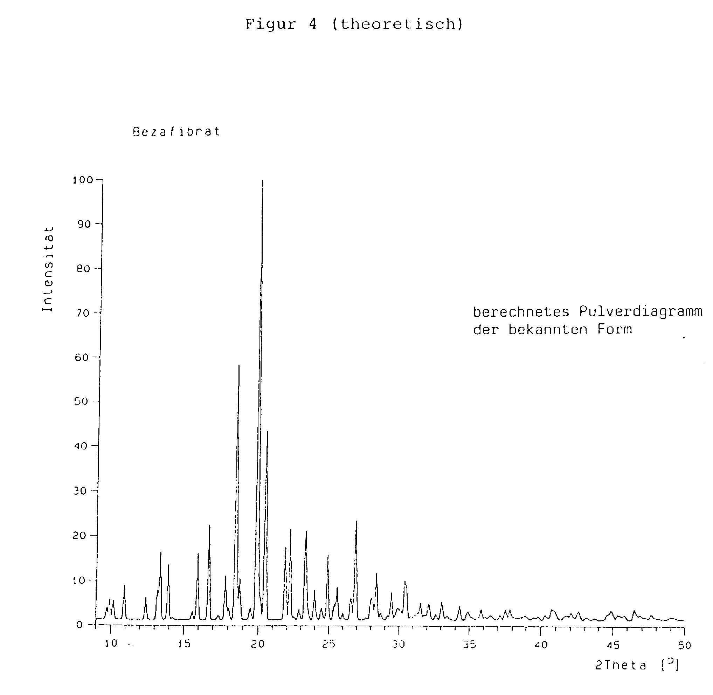

Zusätzlich wurde aufgrund der Literaturdaten für die Kristallstruktur der bekannten orthorhombischen Form (K. Djinovic, loc. cit.) ein theoretisches Pulverdiagramm berechnet, das in Figur 4 wiedergegeben ist und dessen Reflexe in Lage und Intensitäten gut mit dem für die bekannte orthorhombische Form gemessenen Diagramm übereinstimmen.

Die erfindungsgemäße β-Form des Bezafibrats zeigt mit 188°C einen etwas höheren Schmelzpunkt als die bisher bekannte orthorhombische Form.

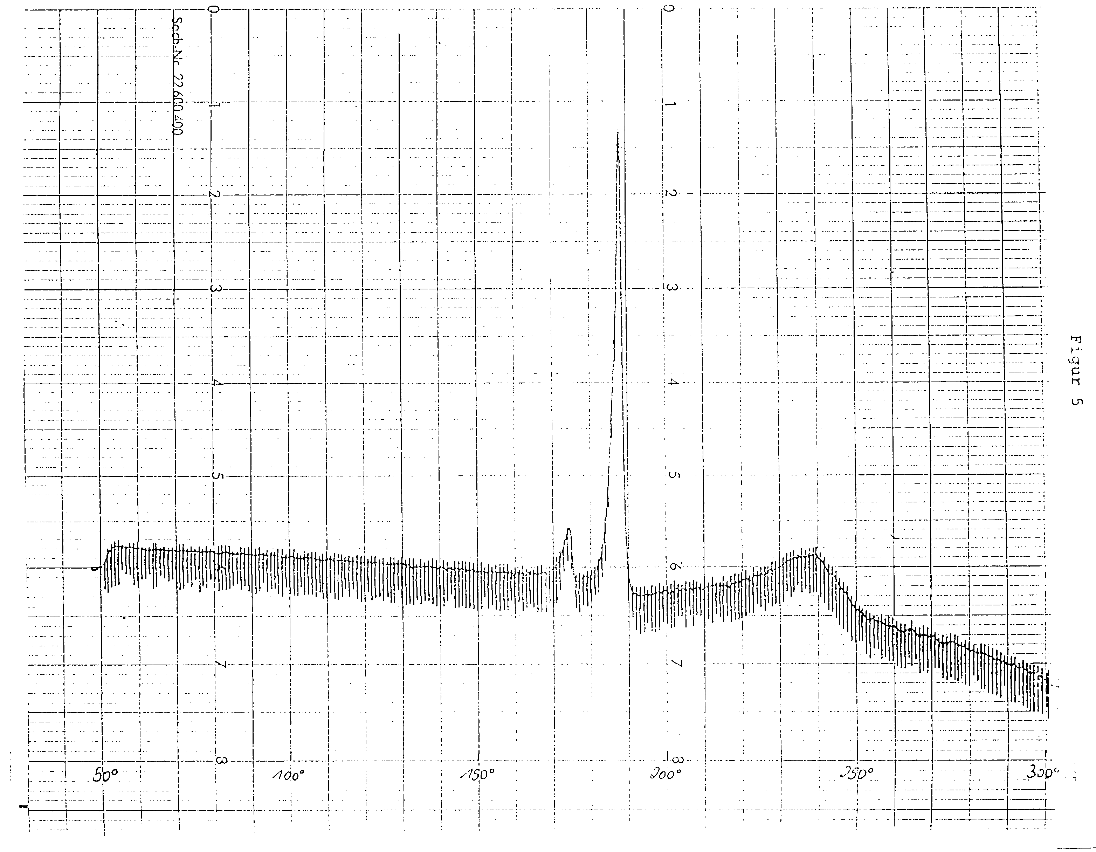

Das DTA-Spektrum der erfindungsgemäßen β-Form, aufgenommen auf einem Mettler FP 85 im Bereich von 50 - 300°C mit einer Heizrate von 2°C/min, ist in Figur 5 wiedergegeben. Neben dem starken endothermen Peak des Schmelzvorgangs bei 188°C tritt noch ein schwacher endothermer Peak bei 173°C auf.

Die erfindungsgemäße kristalline β-Form des Bezafibrats kann nach der Erfindung durch ein Verfahren hergestellt werden, das dadurch charakterisiert ist, daß Bezafibrat aus Gemischen aus Wasser und einem aliphatischen Keton, das zusätzlich bezogen auf das Gesamtvolumen 1 bis 10 Vol.-%, vorzugsweise 4 bis 10 Vol.-%, eines niederen Alkohols enthält, kristallisiert wird. Beispiele für geeignete aliphatische Ketone sind Methylethylketon, Diethylketon und Methylisobutylketon, wobei Methylethylketon bevorzugt wird. Das Volumenverhältnis von Wasser zu dem aliphatischen Keton kann in weiten Grenzen variiert werden. So sind beispielsweise Gemische mit einem Volumenverhältnis von 10 Vol.-% Wasser und 90 Vol.-% Keton bis zu 90 Vol.-% Wasser und 10 Vol.-% Keton geeignet, wobei Gemische mit gleichen Volumenteilen Wasser und Keton, d.h. 50 Vol.-% Wasser und 50 Vol.-% Keton, speziell im Fall von Methylethylketon bevorzugt werden.

Das Gemisch enthält weiterhin bezogen auf das Gesamtvolumen 1 bis 10 Vol.-%, vorzugsweise 4 bis 10 Vol.-%, eines niederen Alkohols als dritte Komponente. Beispiele für den niederen Alkohol sind Methanol, Ethanol, 1-Propanol, Isopropanol, 1-Butanol, 2-Butanol oder Isobutanol. Dabei werden die Butanole, insbesondere 1-Butanol, wegen ihres Siedepunktes bevorzugt. Bei Verwendung von 1-Butanol wird ein Volumenanteil von 4 bis 5 Vol.-% bevorzugt.

Bei Durchführung des erfindungsgemäßen Verfahrens kann im Labormaßstab die allgemein bekannte Verfahrensweise angewendet werden. So wird das Bezafibrat in dem jeweils verwendeten Lösungsmittelgemisch bei dessen Rückflußtemperatur in Lösung gebracht. Dann wird die erfindungsgemäße β-Form durch Abkühlen auf Raumtemperatur oder Temperaturen bis 0°C auskristallisiert. Die Abkühlungszeit beträgt 4 bis 12 Stunden. Bei der technischen Herstellung wird es bevorzugt, eine heiße Lösung von Bezafibrat in dem oben beschriebenen Lösungsmittelgemisch in eine in einem zweiten Kessel vorgelegte Suspension einer kleinen Menge der erfindungsgemäßen β-Form des Bezafibrats in dem oben beschriebenen Lösungsmittelgemisch, die auf 10 bis 30°C, beispielsweise 20°C, gehalten wird, zu geben. Durch die vorgelegte Menge von Impfkristallen wird eine schnelle und von der Kristallgröße reproduzierbare Kristallisation erreicht. Die Abkühlungszeit beträgt in diesem Fall 0,5 bis 2 Stunden.

Die erfindungsgemäße neue β-Form des Bezafibrats hat aufgrund ihrer kristallinen Eigenschaften überraschende Vorteile sowohl im Hinblick auf ihre Synthese als auch für die galenische Verarbeitung zu festen Arzneiformen. Während die bekannte orthorhombische Modifikation in Form von feinen, verfilzten Nadeln kristallisiert - die schlecht zu verarbeiten sind - und mit 0,26 g/ml eine geringe Schüttdichte aufweist, kristallisiert die erfindungsgemäße β-Form in Form von grobkristallinen, kubischen Kristallen und weist mit 0,44 g/ml eine fast doppelt so hohe Schüttdichte auf. Daher zeigt die erfindungsgemäße β-Form eine sehr viel bessere Filtrierbarkeit, die sich z.B. in deutlich verkürzten Zentrifugationszeiten äußert. Dies hat auch Einflüsse auf die Qualität des erhaltenen Produkts, da sich die Filterkuchen besser von Mutterlauge freiwaschen lassen und auch die Gehalte des Produkts an Restlösungsmitteln geringer sind.

Bei der pharmazeutischen Herstellung fester Arzneiformen weist die erfindungsgemäße β-Form des Bezafibrats Vorteile durch ihr besseres Fließverhalten und die im Vergleich zur bekannten Form deutlich höheren Schütt- und Stampfdichten auf. Diese Eigenschaften erlauben eine wirtschaftlichere Herstellung z.B. durch höhere Chargengrößen und kürzere Prozeßzeiten. Auch sind z.B. die erhaltenen Tabletten, bedingt durch den signifikanten Unterschied in der Dichte und angesichts des hohen Wirkstoffanteils, deutlich kleiner.

Die nachfolgenden Beispiele erläutern die Erfindung.

### Beispiel 1

### Herstellung der erfindungsgemäßen β-Form von Bezafibrat

20 g Bezafibrat, erhalten nach dem Verfahren der DE-PS 21 49 070, Beispiel 2a, werden in 200 ml Wasser suspendiert, und die Suspension wird auf Rückflußtemperatur erhitzt. Dann wird in der Siedehitze ein Gemisch aus 200 ml Methylethylketon und 20 ml 1-Butanol zugegeben. Dabei geht der Feststoff vollständig in Lösung. Die erhaltene Lösung wird unter Rühren langsam (z.B. über Nacht) auf Raumtemperatur abkühlen gelassen und weitere 2 Stunden bei Raumtemperatur gerührt. Die erhaltenen groben Kristalle werden abgesaugt, mit 2 x 20 ml Wasser gewaschen und im Vakuum bei 80°C für 2 Stunden getrocknet. Es werden 16 g farblose Kristalle der erfindungsgemäßen β-Form des Bezafibrats erhalten, die das FT-IR-Spektrum gemäß Figur 1 zeigen.

### Beispiel 2

In einem 250-1-Kessel werden 20 l Wasser, 120 l Methylethylketon und 12 l 1-Butanol vorgelegt, und in das Gemisch werden 40 kg Bezafibrat-Rohprodukt, erhalten nach dem Verfahren der DE-PS 21 49 070, Beispiel 2a, eingetragen. Das Gemisch wird zur Lösung auf 60°C erhitzt und mit 1 kg Aktivkohle und 1 kg Hyflo-Supercel® (Filtrationshilfsmittel) versetzt.

In einem zweiten Kessel werden 4,5 kg der gemäß Beispiel 1 erhaltenen β-Form von Bezafibrat in 2,5 l Wasser, 13 l Methylethylketon und 1 l 1-Butanol vorgelegt und auf 0°C gekühlt.

Die 60 bis 65°C warme Lösung aus dem ersten Kessel wird dann über ein Druckfilter langsam unter Kühlung zu den vorgelegten Impfkristallen im zweiten Kessel gedrückt, wobei die Temperatur des resultierenden Gemisches bei 30 bis 35°C gehalten halten wird. Danach wird auf 0 bis 5°C gekühlt, und der Kristallbrei wird abgesaugt. Nach Waschen mit 2 x 20 l Wasser erhält man 37 kg der erfindungsgemäßen β-Form des Bezafibrats, die durch das FT-IR-Spektrum gemäß Figur 1 charakterisiert ist.

## Patentansprüche

1. Neue, kristalline β-Form von 2-[4-[2-(4-Chlorbenzoylamino)ethyl]phenoxy]-2-methylpropionsäure, **gekennzeichnet** durch ihr FT-Infrarotspektrum in Kaliumbromid mit Absorptionsbanden bei folgenden Wellenzahlen:
| | |
|---|---|
| 3389 cm⁻¹ | 847 cm⁻¹ |
| 1740 cm⁻¹ | 821 cm⁻¹ |
| 1328 cm⁻¹ | 618 cm⁻¹ |
| | 606 cm⁻¹ |

## Claims

1. New, crystalline β-form of 2-[4-[2-(4-chlorobenzoylamino)ethyl]phenoxy]-2-methylpropionic acid, characterised by its FT infrared spectrum in potassium bromide with absorption bands at the following wave numbers:
| | |
|---|---|
| 3389 cm⁻¹ | 847 cm⁻¹ |
| 1740 cm⁻¹ | 821 cm⁻¹ |
| 1328 cm⁻¹ | 618 cm⁻¹ |
| | 606 cm⁻¹ |

## Revendications

1. Nouvelle forme cristalline β de l'acide 2-[4-[2-(4-chlorobenzoylamino)éthyl]phénoxy]-2-méthylpropionique,
caractérisée par son spectre infrarouge FT dans du bromure de potassium avec des bandes d'absorption aux nombres d'onde suivants :
| | |
|---|---|
| 3389 cm⁻¹ | 847 cm⁻¹ |
| 1740 cm⁻¹ | 821 cm⁻¹ |
| 1328 cm⁻¹ | 618 cm⁻¹ |
| | 606 cm⁻¹. |
